(19) Europäisches Patentamt
European Patent Office
Office européen des brevets

(11) Publication number: **0 091 026**
**B1**

(12) # EUROPEAN PATENT SPECIFICATION

(45) Date of publication of patent specification: **11.02.87**

(51) Int. Cl.$^4$: **C 12 Q 1/60,** C 12 Q 1/46

(21) Application number: **83102958.2**

(22) Date of filing: **24.03.83**

(54) **Improved time-stable liquid cholesterol assay compositions.**

(30) Priority: **02.04.82 US 364899**

(43) Date of publication of application:
**12.10.83 Bulletin 83/41**

(45) Publication of the grant of the patent:
**11.02.87 Bulletin 87/07**

(84) Designated Contracting States:
**AT BE CH DE FR GB IT LI LU NL SE**

(56) References cited:
**EP-A-0 024 578**
**GB-A-1 479 994**
**US-A-4 226 713**

**CLINICAL CHEMISTRY, vol. 25, no. 6, june 1979, Easton, Pennsylvania, USA; P.J.G. DAWSON et al. "Enzymic Assay of Total Cholesterol Involving Chemical or Enzymic Hydrolysis - A Comparison of Methods", pages 976-984**

(73) Proprietor: **Modrovich, Ivan Endre**
**829 Via Alondra**
**Camarillo California 93010 (US)**

(72) Inventor: **Modrovich, Ivan Endre**
**829 Via Alondra**
**Camarillo California 93010 (US)**
Inventor: **Caris, Karen Rae**
**1399 North Lucero Street**
**Camarillo California 93010 (US)**

(74) Representative: **Patentanwälte Grünecker, Dr. Kinkeldey, Dr. Stockmair, Dr. Schumann, Jakob, Dr. Bezold, Meister, Hilgers, Dr. Meyer-Plath**
**Maximilianstrasse 58**
**D-8000 München 22 (DE)**

**EP 0 091 026 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

Background of the invention

It has been known to determine cholesterol in sera by the use of assay compositions based on cholesterol oxidase, presently from a microbial source. The reaction involved is:

$$\text{cholesterol}+O_2 \xrightarrow{\text{cholesterol oxidase}} \text{cholest-4-en-3-one}+H_2O_2 \quad (1)$$

For total cholesterol determination, bound cholesterol may be released by the inclusion of cholesterol esterase which yields cholesterol by the reaction:

$$\text{cholesterol ester}+H_2O \xrightarrow{\text{cholesterol esterase}} \text{cholesterol}+RCOOH \quad (2)$$

The amount of cholesterol can be assayed by measuring the amount of oxygen consumed, the amount of cholest-4-en-3-one formed, or the amount of hydrogen peroxide formed. A preferred way is to determine the amount of hydrogen peroxide formed by use of a chromogen system. A preferred chromogen system is one based on the presence of peroxidase from a horseradish source, phenol and antipyrine involving the reaction:

$$2H_2O_2+\text{4-aminoantipyrine}+\text{phenol} \xrightarrow{\text{peroxidase}} \text{quinoneimine dye}+4H_2O \quad (3)$$

While most assay systems based on cholesterol oxidase can be made functional as prepared, they are prone to rapid degradation. As a consequence, the art early on lyopholized (freeze-dried) the composition for reconstitution at the time of use. Lyopholization is expensive and suffers from inaccuracy.

A need was recognized to provide a liquid assay system of controlled composition which would have an adequate shelf life for marketing purposes. As invented and described by one of us, and disclosed in EPC Application 80.104.568.3, filed 1 August, 1980, and published as EP—A—24578, it was found that the presence of a material quantity, e.g., up to 50 percent by volume, of a polyhydroxy compound such as glycerol would induce long shelf life to a liquid assay composition. The invention enabled precise quality control to be exercised over the composition of the system, and enabled total reliability of the assay system as a tool. The system was formulated as a concentrate. Shelf life of the concentrate was more than adequate for industrial use and provided levels of stability theretofore unknown in the art.

The polyhydroxy compound, while functional to stabilize the system against degradation, increases costs and, unless proper housekeeping procedures are followed, contaminates apparatus, affecting other tests, particularly triglyceride analysis.

A desire has existed, therefore, for a liquid assay system which did not yield in performance, which could be sold as a single formulation for use as is without dilution and yet have an adequate shelf life to satisfy marketing requirements.

Summary of the invention

It has now been found that utilizing basic constituents normally present in a cholesterol assay system, but exercising exacting control over concentration of bile acid or salts thereof, nonionic surfactant and buffer, as well as pH, one can formulate a stable cholesterol assay composition which does not require a polyhydroxy compound and yet exhibits projected shelf lives of 18 months or more at 4°C, and when used with a chromogen system, rapid completion times.

The base solution employed is an aqueous solution of at least one acidic compound which is a bile acid or salt thereof, present in a concentration of up to about 5 mM, preferably from about 0.2 to about 5 mM; a nonionic surfactant, preferably propylene glycol p-isoocytylphenyl ether, present in a concentration of from about 0.15 to about 1.5 percent volume by volume, preferably from about 0.2 to about 0.6 percent volume by volume, from 0 to 65 mM of a buffer, preferably from 0.5 to 50 mM, and more preferably from 0.5 to 30 mM, the preferred buffer being potassium dihydrogen phosphate ($KH_2PO_4$); and cholesterol oxidase in a concentration of at least 0.02 KIU/l, preferably at least 0.05 KIU/l, the solution having a pH of from about 5.5 to about 8.5, preferably from 6 to about 7.5.

Where it is desired to assay for total cholesterol, there is included in the composition a microbial cholesterol esterase present in a concentration of at least 0.07 KIU/l, preferably at least about 0.1 KIU/l.

The preferred composition is one containing a chromogen system for determination of hydrogen peroxide. The chromogen system preferably comprises phenol in a concentration of from 8 to about 35 mM; 4-aminoantipyrine in a and peroxidase in a concentration sufficient to enable completion of a chromogen reaction, i.e., development of the pink quinoneimine dye to an intensity quantitative to hydrogen peroxide formed. For commercial practicality, they are provided in quantities sufficient to enable

completion of the reaction within 10 minutes at 37°C. Preferably, the peroxidase is provided in a concentration of at least 30 KIU/l, and 4-aminoantipyrine to a concentration of about 0.3 mM.

There is preferably included in the composition a bacteriocide, with the preferred bacteriocide being 2,4 dichlorophenol, present in a concentration of up to about 1 mM, preferably from about 0.4 to about 0.6 mM.

The compositions prepared in accordance with the instant invention are stable for at least 3 days at 41°C, which is equivalent to a projected shelf life of 18 months at 4°C or about 6 months at ambient temperature (25°C).

When a chromogen system is employed, completion of reaction preferably occurs within 10 minutes or less at 37°C, with a color stability of at least an additional 30 minutes.

The products are prepared by first forming an aqueous solution to which there is provided buffer, bile acid or salts thereof, and surfactant. Phenol, dichlorophenol and 4-aminoantipyrine are added as required. This base composition is adjusted, if required, to an acceptable pH range by addition of a suitable acid or base.

There is separately formed an aqueous solution containing the nonionic surfactant and the enzymes which are added. The base solution and the solution of the enzymes are then combined to form a net solution.

Detailed description

According to the present invention there is provided an assay solution for the determination of cholesterol in the liquids, including sera, and which display a protracted shelf life, i.e., a shelf life of about 18 months or more at 4°C (refrigeration conditions). Long shelf life is primarily the result of control over concentration of buffer employed.

A stable cholesterol assay composition of the instant invention comprises an aqueous solution of at least one acidic compound which is a bile acid and/or a salt of a bile acid, the total of said acidic compound being present in an amount of up to about 5 mM, preferably from about 0.2 to about 5 mM; a nonionic surfactant present in a concentration of from about 0.15 to about 1.5 percent volume by volume, preferably from about 0.2 to about 0.6 percent volume by volume; a buffer in a concentration of from 0 to about 65 mM, preferably from about 0.5 to about 50 mM; cholesterol oxidase in a concentration of at least about 0.02 KIU/l, preferably at least 0.05 KIU/l, the solution having a pH of from about 5.5 to about 8.5.

For total cholesterol assay there is included microbial cholesterol esterase present in a concentration of at least about 0.07 KIU/l, preferably at least about 0.1 KIU/l.

The preferred cholesterol assay composition includes a chromogen system for determination of hydrogen peroxide.

More particularly, the preferred chromogen cholesterol assay solutions of the instant invention provide, on a perliter basis, phenol in a concentration of from about 8 to about 35 mM, preferably from about 15 to about 20 mM; bile acid and/or a salt of bile in a total amount up to about 5.0 mM, preferably from about 0.2 to about 5 mM; a nonionic surfactant, preferably polyethylene glycol p-isooctylphenyl ether (TRITON X-100), in a concentration of from about 0.15 to about 1.5 percent by volume, preferably from 0.2 to about 0.6 percent volume by volume; a buffer in a concentration of from 0 to 65 mM, preferably from about 0.5 to about 50 mM; cholesterol oxidase in a concentration of at least 0.02 KIU/l; peroxidase, preferably in a concentration of at least about 30 KIU/l; and, if present, cholesterol esterase in a concentration of at least 0.07 KIU/l, preferably at least about 0.1 KIU/l. Peroxidase and 4-aminoantipyrine are provided in an amount sufficient to enable quantitative colormetric determination of the amount of hydrogen peroxide formed from oxidation of cholesterol. It is preferred that this occur within a 10-minute completion time at 37°C. To this end, it is preferred that 4-aminoantipyrine be present in a concentration of about 0.3 mM. An acceptable range is from about 0.2 mM to about 0.35 mM. If too much or too little 4-aminoantipyrine is present, the reaction will not achieve completion, if at all, in the desired time span.

It is preferred to include in the system a bacteriocide. The preferred bacteriocide is dichlorophenol, and may be provided in a concentration of up to 0.75 mM, preferably from about 0.4 to about 0.5 mM.

The buffer is provided as required, and can be inorganic or organic in nature. Phosphates are preferred. The presently preferred buffer is potassium dihydrogen phosphate ($KH_2PO_4$).

The preferred acidic compound is cholic acid or a metal salt thereof. The presently preferred compound is sodium cholate.

The chromogen cholesterol assay compositions of the instant invention display the ability to recover, i.e., detect, cholesterol; and preferably provide an assay completion time within 10 minutes at 37°C to a pink color, the developed intensity of which is stable for at lesat 30 additional minutes. The compositions have a projected stability of at least 18 months at 4°C, or a shelf life of about 6 months at room temperature, as determined by a requirement that they are stable for at least 3 days at 41°C. The chromogen assay systems of the invention are used as such and do not require dilution.

In the chromogen cholesterol assay compositions of the instant invention, a lower level of phenol concentration defines the point at which the system will lose stability, and the upper concentration defines the point at which phenol has reached a concentration where there may be an adverse effect upon color.

Besides being functional as a bacteriocide, dichlorophenol may help speed color development, and therefore is a highly desirable constituent, independent of its bacteriocide function.

The upper level of buffer concentration is critical. If the concentration is too high, completion time will be too slow, giving unreliable results and, quite unexpectedly, there will be an adverse effect on shelf life.

A bile acid or a bile salt is essential. In the absence thereof, the system fails to recover cholesterol. By contrast, at a concentration above about 5 mM, completion times are too long for commercial utility.

The nonionic surfactant has been observed to activate the enzymes, particularly cholesterol esterase. In its absence, reaction time is too long, and if present in too high a concentration will result in foaming and may have an adverse effect on viscosity.

The cholesterol oxidase used in the practice of this invention is currently of a microbial nature. The presently utilized cholesterol oxidase is that manufactured and sold by Whatman Biochemicals, Inc., of England. It has been observed that cholesterol oxidase of the Brevi bacterium is non-functional. Cholesterol esterase is from any microbial source, and that used is manufactured and sold by Kyowa Hakko Kogyo Company, Ltd., of Japan, understood to be produced from the microorganism pseudomonas fluorescens, ATCC 1126. The peroxidase used is, conveniently, horseradish peroxidase.

The products are prepared by first forming an aqueous solution to which there is provided buffer, bile acid or salts thereof, and surfactant. Phenol, dichlorophenol and 4-aminoantipyrine are added as required. This base composition is adjusted, if required, to an acceptable pH range by addition of a suitable acid or base.

There is separately formed an aqueous solution containing the nonionic surfactant and the enzymes which are added. The base solution and the solution of the enzymes are then combined to form a net solution.

The following is the presently preferred chromogen composition, based on the total volume of 1 liter:

| Component | Concentration |
|---|---|
| Phenol | 17 mM |
| $KH_2PO_4$ | 12.5 mM |
| 2,4 dichlorophenol | 0.49 mM |
| 4-aminoantipyrine | 0.295 mM |
| Cholic acid | 2.3 mM |
| Cholesterol oxidase | 0.05 KIU/l |
| Cholesterol esterase | 0.1 KIU/l |
| Peroxidase | 30 KIU/l |
| Triton X-100 | 0.4±.2 v/v |

Without limiting, the following Examples and Controls illustrate the various parameters associated with the compositions of the instant invention.

Example 1

There was formulated a cholesterol assay system by forming a clear base solution of the following composition:

| Component | Concentration |
|---|---|
| Water (triple-distilled deionized) | 0.955 liter |
| Triton X-100 (10% v/v solution) | 32.0 ml |
| $KH_2PO_4$ | 12.5 mM |
| 2,4 dichlorophenol | 0.49 mM |
| 4-aminoantipyrine | 0.3 mM |
| Phenol | 17.0 mM |
| Sodium Cholate | 2.3 mM |
| pH | 7.0 |

A clear enzyme solution was formed by addition to 10 ml of an aqueous solution containing Triton X-100, sufficient cholesterol oxidase to provide cholesterol oxidase in a net solution of 0.1 KIU/l, cholesterol esterase in an amount sufficient to provide in the net solution a cholesterol esterase concentration of 0.2 KIU/l, and peroxidase in an amount sufficient to provide in the net solution peroxidase in a concentration of 30 KIU/l.

The enzyme solution was combined with the base solution. The solution recovered cholesterol in an assay with less than a 10-minute completion time at 37°C. The color formed had a stability of greater than 30 minutes, and had a lifetime of in excess of 3 days at 41°C, which is an equivalent of a shelf life of 18 months at 4°C and about 6 months at room temperature.

Detailed studies were made of variations of the assay composition prepared according to Example 1. The parameters varied were buffer concentration, pH, cholic acid concentration and nonionic detergent concentration. For purposes of all Examples and Controls, the following meanings or codes universally apply:

1 = No change
$A_i'$ = Initial absorbance at 500 nm at 37°C must be less than or equal to 0.15 for a pass
2 = A control manufactured and sold by Beckman Instruments, Inc. that is specific to cholesterol
3 = Mean or principle assigned value (PAV) to the control times 1 or the factor shown
4 = Lot number of Control
5 = A control manufactured and sold by Beckman Instruments, Inc. for multiple assay, including cholesterol
6 = A cholesterol control manufactured and sold by New England Reagent Laboratories. Cholesterol concentration was 200 mg/dl
T = TRITON X-100=a polyethylene glycol p-isooctylphenyl ether having an average formula of $C_{34}H_{62}O_{11}$ and a formula weight of 646, manufactured and sold by Eastman Chemicals

Completion times are for Beckman references at a cholesetrol concentration of 600 mg/dl.

Color stability is for a cholesterol concentration of 50 mg/dl (low) and/or 500 mg/dl (high). Numerical value given is % change at the time stated.

One or more of the following constitutes failure:

a) no recovery (detection) of cholesterol;

b) greater than 10 minutes completion time at 37°C; this is failure on the basis that longer completion times are commercially unacceptable;

c) color stability for less than 30 minutes beyond completion time; and/or

d) stability for less than three days at 41°C (stressed).

Failure is also considered to occur if initial absorbent $A_i'$ is greater than 0.15 and cholesterol recovery (level detected) is not within ±5% of sample.

Examples 2—7 and Controls A—C

Buffer concentration

The solution, formulated in accordance with Example 1, was modified in respect to $KH_2PO_4$ concentration. All other constituents were kept constant.

Table I compares performance as formulated (fresh) and after stressed by being heated to 41°C for the time specified in the Table. Controls A, B and C failed because of long completion times after stress.

TABLE I

| Example or control | Reagent Condition | pH | Color stability Low | Color stability High | Buffer concentration mM/L | Completion time minutes | $A_i'$ | 2.5X Beckman reference[2] 587.5 C-011044[4] | Decision I[5] 125[3] C-007014[4] | Decision II[5] 139[3] C-104032[4] | Decision III[5] 211[3] C-007016[4] | 2X Decision III[5] 422[3] | 3X Decision III[5] 633[3] |
|---|---|---|---|---|---|---|---|---|---|---|---|---|---|
| Example 2 | Fresh | ALL ~ 7.0 | 0' | | 0.0 | 3 | .008 | — | — | — | — | — | — |
| | 82 hrs. at 41°C | | | 2.2% | | 6 | .028 | 615 | 144 | 144 | 229 | 427 | 630 |
| Example 3 | Fresh | | 0 | | 1.0 | 3 | .008 | 604 | 133 | 146 | 225 | 452 | 647 |
| | 82 hrs. at 41°C | | | 4.0% | | 7 | .028 | 603 | 137 | 144 | 225 | 440 | 646 |
| Example 4 | Fresh | | 0 | | 5.0 | 3 | .008 | 593 | 131 | 142 | 224 | 442 | 653 |
| | 82 hrs. at 41°C | | | 2.2% | | 7 | .031 | 599 | 133 | 145 | 220 | 442 | 654 |
| Example 5 | Fresh | | 0 | | 12.5 | 3 | .008 | 590 | 130 | 144 | 222 | 443 | 652 |
| | 82 hrs. at 41°C | | | 1.5% | | 7 | .035 | 598 | 132 | 144 | 222 | 441 | 653 |
| Example 6 | Fresh | | 0 | | 25.0 | 3 | .008 | 589 | 131 | 143 | 222 | 441 | 643 |
| | 82 hrs. at 41°C | | | 0 | | 8.5 | .038 | 604 | 132 | 144 | 222 | 439 | 652 |
| Example 7 | Fresh | | 0 | | 50.0 | 3 | .008 | 588 | 131 | 146 | 222 | 442 | 652 |
| | 82 hrs. at 41°C | | | 0 | | 8.5 | .040 | 609 | 130 | 144 | 220 | 439 | 659 |
| Control A | Fresh | | — | | 75.0 | 3 | .008 | | | | | | |
| | 82 hrs. at 41°C | | | — | | 15 | .045 | | | | | | |
| Control B | Fresh | | — | | 100.0 | 5 | .008 | | | | | | |
| | 48 hrs. at 41°C | | | — | | 13 | .038 | | | | | | |
| Control C | Fresh | | — | | 200.0 | 4-5 | .008 | | | | | | |
| | 48 hrs. at 41°C | | | — | | 25 | .053 | | | | | | |

Examples 8, 9 and Controls D—I

Evaluation of pH

Using the assay solution prepared according to Example 1, pH was changed using HCl or NaOH to determine its effect on performance. Using the same references of Examples 2—7, the results are shown in Table II.

Failures were due to too long a completion time. Controls D, H and I failed as prepared. Controls E and F failed after stressed for 48 hours, while Control G failed after stressed after 82 hours. Color stability was after 45 minutes.

TABLE II

| Example or control | Reagent Condition | pH | Color Stability Low | Color Stability High | Completion time minutes | $A_i'$ | Beckman reference[2] 2.5X C-011044[4] | Decision I[5] 125[3] C-007014[4] | Decision II[5] 139[3] C-104032[4] | Decision III[5] 211[3] C-007016[4] | 2X decision III[5] 422[3] | 3X decision III[5] 633[3] |
|---|---|---|---|---|---|---|---|---|---|---|---|---|
| Control D | Fresh | 3.5 | | | 14 | .008 | | | | | | |
| Control E | Fresh | 4.0 | | | 10 | .008 | | | | | | |
| | 48 hrs. at 41°C | | | | >30 | .188 | | | | | | |
| Control F | Fresh | 5.0 | | | 9—3/4 | .008 | | | | | | |
| | 48 hrs. at 41°C | | | | FAIL | .072 | | | | | | |
| Example 8 | Fresh | 6.0 | 0 | | 3-1/2 | .008 | 592 | 131 | 148 | 225 | 450 | 667 |
| | 48 hrs at 41°C | | | | 5 | .026 | | | | | | |
| | 82 hrs at 41°C | | | 0 | 7-1/2 | .036 | 593 | 134 | 149 | 229 | 453 | 674 |
| Example 9 | Fresh | 7.0 | 3.5% | | 3 | .008 | 589 | 130 | 143 | 222 | 441 | 655 |
| | 48 hrs at 41°C | | | | 5 | .024 | | | | | | |
| | 82 hrs at 41°C | | | 0 | 6-1/2-7 | .032 | 594 | 127 | 145 | 226 | | 661 |
| Control G | Fresh | 8.0 | | | 5-3/4 | .008 | | | | | | |
| | 48 hrs at 41°C | | | | 7 | .046 | | | | | | |
| | 82 hrs at 41°C | | | | 11 | .065 | | | | | | |
| Control H | Fresh | 9.0 | | | >16 | .008 | | | | | | |
| Control I | Fresh | 9.5 | | | >15 | .008 | | | | | | |

Examples 10—14 and Controls J, K

Cholic acid effect

Using the assay composition of Example 1, cholic acid concentration was varied, with all other factors kept constant. The results are shown in Table III. Control J failed because the system was turbid, and collapsed when applied to human sera. Control K failed because completion time in human sera was too long, even on stress of the solution by heating to 41°C for 72 hours. Color stability was after 75 minutes at 37°C.

TABLE III

| Example or control | Cholic acid g/l | Cholic acid Cond. | pH | Color stability Low | Color stability High | Completion time sera minutes | Completion time minutes | $A_i$ | NERL[6] 200 mg/dl | Decision I[5] 124[3] C-007014[4] | Decision II[5] 134[3] C-104032[4] | Decision III[5] 211[3] C-007016[4] | 2X Decision III[5] 422[3] | 3X Decision III[5] 633[3] |
|---|---|---|---|---|---|---|---|---|---|---|---|---|---|---|
| Control J | 0.0 | Fresh | ALL ~ 7.0 | | | | ~5—6 | | | | | | | |
| | | 72 hrs. at 41°C | | 0 | <1% | (Turbidity) COLLAPSE | 6,6 | .045 | 200 | 114 | 127 | 192 | 278 | 559 |
| Example 10 | 0.1 | Fresh | | | | | | | | | | | | |
| | | 72 hrs. at 41°C | | | | 7 | 7 | .037 | | | | | | |
| Example 11 | 0.3 | Fresh | | | | | | | | | | | | |
| | | 72 hrs. at 41°C | | | | 6-1/2 | 6 | .033 | | | | | | |
| Example 12 | 0.75 | Fresh | | | | | | | | | | | | |
| | | 72 hrs. at 41 C | | | | 6 | 6 | .032 | | | | | | |
| Example 13 | 1.0 | Fresh | | | | | ~5—6 | | | | | | | |
| | | 72 hrs. at 41°C | | 0 | <2% | 5 | 7 | .034 | | 122 | 135 | | | |
| Example 14 | 1.5 | Fresh | | | | | | | | | | | | |
| | | 72 hrs. at 41 C | | | | 5-3/4 | 7 | .035 | | | | | | |
| Control K | 2.0 | Fresh | | | | | ~6—7 | | | | | | | |
| | | 72 hrs. at 41 C | | | <1% | 10-1/2 | 8-1/2 | .036 | | 123 | 138 | 209 | 411 | 614 |

Examples 15—20 and Controls L, M

Nonionic surfactant

Since the enzymes require some nonionic surfactant in (Triton X-100) for initial enzyme stability, "0" in Control L of Table IV means a concentration on a volume basis of 6 parts per 10,000 parts. Completion times for non-sera were at a cholesterol concentration of 567.5 mg/dl. Cholesterol concentration of the sera used for sera completion time was 650 mg/dl. Control L failed because of too long a completion time in sera.

TABLE IV

| Example or control | T* | Cond. | pH | Color stability Low | Color stability High | Completion time sera minutes | Completion time minutes | $A_i$ | NERL 200 mg/dl | SERA | Decision I[5] 123[3] C-007014[4] | Decision III[5] 211[3] C-007016[4] | 2X decision III[5] 422[3] | 3X Decision III[5] 633[3] |
|---|---|---|---|---|---|---|---|---|---|---|---|---|---|---|
| Control L | 0.0 | Fresh | 7.02 | | | | 7 | | | | | | | |
| | | 72 hrs. at 41°C | 7.01 | 2% | <1% | 20 | 9 | .031 | | 263 | 124 | 209 | 408 | 603 |
| Control M | 0.11 | Fresh | 7.02 | | | | 7.5 | | | | | | | |
| | | 72 hrs. at 41°C | 7.01 | 2% | <1% | | 10 | | | | | | | |
| Example 15 | 0.21 | Fresh | 7.02 | | | | 7 | | | | | | | |
| | | 72 hrs. at 41°C | 7.01 | 2% | <1% | | 9.5 | | | | | | | |
| Example 16 | 0.29 | Fresh | 7.02 | | | | 8 | | | | | | | |
| | | 72 hrs. at 41°C | 7.01 | 2% | <1% | | 9 | | | | | | | |
| Example 17 | 0.38 | Fresh | | | | 3.5 | 7.5 | | | | | | | |
| | | 72 hrs. at 41°C | | 0% | <1% | 5.25 | 8, 7.5 | | | 250 | 122 | 206 | 407 | 616 |
| Example 18 | 0.70 | Fresh | 7.02 | | | | 8 | | | | | | | |
| | | 72 hrs. at 41°C | 7.01 | 0% | <1% | 6.5 | 8, 8.25 | | | 252 | 121 | 206 | 413 | 613 |
| Example 19 | 1.02 | Fresh | 7.02 | | | 3.5 | 8 | | | | | | | |
| | | 72 hrs. at 41°C | 7.01 | | | | | | | | | | | |
| Example 20 | 1.34 | Fresh | 7.02 | | | | 8 | | | | | | | |
| | | 72 hrs. at 41°C | 7.01 | | 0% | | | | | | | | | |

*Triton X-100 Concentration Percent Volume by Volume

Where, in the above Controls, failure is due to too long a completion time, as opposed to inability to recover cholesterol after stress, it is considered only to define a composition considered to have a commercial lack of utility, as completion time is important. Therefore, the specification of the claims is oriented to a commercial product of short completion times. It will be considered, however, to be in the invention a system having longer completion times, provided they have adequate shelf life.

**Claims for the Contracting States: BE, CH, DE, FR, GB, IT, LI, LU, NL, SE**

1. A stable cholesterol assay composition which comprises an aqueous solution of:

a) at least one acidic compound selected from a bile acid and a salt of a bile acid, the total of said acidic compound being present in an amount of up to about 5 mM;

b) a nonionic surfactant present in a concentration of from about 0.15 to about 1.5 percent volume by volume;

c) a buffer in a concentration of from 0 to about 65 mM; and

d) cholesterol oxidase in a concentration of at least about 0.02 KIU/l,

said cholesterol assay solution having a pH of from about 5.5 to about 8.5.

2. A stable cholesterol assay composition as claimed in claim 1 which includes microbial cholesterol esterase present in a concentration of at least about 0.07 KIU/l.

3. A stable cholesterol assay composition as claimed in claim 1 or 2 which includes a chromogen system for determination of hydrogen peroxide.

4. A stable cholesterol assay system as claimed in claim 3 in which the chromogen system comprises phenol in a concentration of from about 8 to about 35 mM, and peroxidase and 4-aminoantipyrine in a concentration sufficient to provide a colormetric quantitative determination of the hydrogen peroxide formed from oxidation of cholesterol.

5. A stable cholesterol assay system as claimed in claim 1 or 2 which includes a chromogen system for determining hydrogen peroxide and which comprises phenol in a concentration of from about 8 to about 35 mM, 4-aminoantipyrine in a concentration of from about 0.25 to about 0.35 mM, and peroxidase in a concentration of at least about 30 KIU/l.

6. A stable cholesterol assay composition as claimed in any one of the previous claims which includes a bacteriocide.

7. A stable cholesterol assay composition as claimed in claim 6 which includes 2,4 dichlorophenol in a concentration of up to about 1 mM.

8. A stable cholesterol assay composition as claimed in any one of the previous claims in which the nonionic surfactant is polyethylene glycol p-isooctylphenyl ether.

9. A stable cholesterol assay composition as claimed in any one of the previous claims in which the buffer is potassium dihydrogen phosphate.

10. A stable cholesterol assay composition as claimed in any one of the previous claims in which the acidic compound is a metal salt of cholic acid.

11. A stable total cholesterol chromogen assay composition comprising an aqueous solution having a pH of from about 6.5 to about 8.5 and comprising:

a) phenol in a concentration of from about 8 to about 35 mM;

b) a metal salt of cholic acid present in a concentration of up to about 5 mM;

c) a nonionic surfactant present in a concentration of from about 0.2 to about 1.5 percent volume by volume;

d) a buffer present in a concentration of from 0 to about 65 mM;

e) 4-aminoantipyrine;

f) microbial cholesterol esterase present in a concentration of at least about 0.07 KIU/l;

g) cholesterol oxidase present in a concentration of at least about 0.02 KIU/l; and

h) peroxidase,

the amount of peroxidase and 4-aminoantipyrine being sufficient to enable quantitative determination of the amount of hydrogen peroxide formed from oxidation of cholesterol.

12. A stable total cholesterol chromogen assay composition as claimed in claim 11 in which the buffer is potassium dihydrogen phosphate and pH is from about 6 to about 7.5.

13. A stable total cholesterol chromogen assay composition as claimed in claim 11 or 12 in which the nonionic surfactant is present in a concentration of from about 0.2 to about 0.4 percent volume by volume and is polyethylene glycol p-isooctylphenyl ether.

14. A stable total cholesterol chromogen assay composition as claimed in any one of the previous claims in which peroxidase is present in a concentration of at least about 30 KIU/l and in which 4-aminoantipyrine is present in a concentration of about 0.3 mM.

15. A stable total cholesterol chromogen assay composition comprising an aqueous solution of:

a) phenol in a concentration of about 17 mM;

b) 2,4 dichlorophenol present in a concentration of about 0,5 mM;

c) a metal salt of cholic acid present in a concentration of up to about 5 mM;

d) polyethylene glycol p-isooctylphenyl ether present in a concentration of from about 0.2 to about 0.6 percent volume by volume;

e) $KH_2PO_4$ present in a concentration of about 12.5 mM;
f) peroxidase present in a concentration of about 30 KIU/l;
g) cholesetrol oxidase present in a concentration of at least about 0.05 KIU/l;
h) microbial cholesterol esterase present in a concentration of at least about 0.1 KIU/l; and
i) 4-aminoantipyrene present in a concentration of about 0.3 mM,
said stable total cholesterol chromogen assay composition having a pH of from about 6.0 to about 7.5.

16. A method for preparing a stable cholesterol assay solution which comprises forming a base solution and an enzyme solution, then combining the base solution and the enzyme solution to form a net solution, and in which:

a) the base solution is formed by dissolving in water:

i) at least one acidic compound selected from a bile acid and a salt of a bile acid to provide the total of said acidic compound in the net solution in an amount of up to about 5 mM;
ii) a nonionic surfactant determined to provide an amount in the net solution a nonionic surfactant concentration of from about 0.15 to about 1.5 percent volume by volume;

b) the enzyme solution being formed by dissolving in water containing a portion of the total nonionic surfactant, cholesterol oxidase to provide in the net solution cholesterol oxidase in a concentration of at least about 0.02 KIU/l,

the base solution being adjusted if required to provide a net solution having a pH of from about 5.5 to about 8.5.

17. A method as claimed in claim 16 in which there is added to the enzyme solution microbial cholesterol esterase in an amount to provide in the net solution cholesterol esterase in a concentration of at least about 0.07 KIU/l.

18. A method as claimed in claim 16 or 17 in which there is added to the base solution phenol in an amount sufficient to provide in the net solution phenol in a concentration of from about 8 to about 15 mM, and 4-aminoantipyrine, and in which there is added to the enzyme solution peroxidase, the amount of peroxidase and 4-aminoantipyrine being sufficient in the net solution to enable quantitative colormetric determination of the amount of hydrogen peroxide formed from oxidation of cholesterol.

**Claims for the Contracting State: AT**

1. A method for preparing a stable cholesterol assay solution which comprises forming a base solution and an enzyme solution, then combining the base solution and the enzyme solution to form a net solution, and in which:

a) the base solution is formed by dissolving in water:

i) at least one acidic compound selected from a bile acid and a salt of a bile acid to provide the total of said acidic compound in the net solution in an amount of up to about 5 mM;
ii) a nonionic surfactant determined to provide an amount in the net solution a nonionic surfactant concentration of from about 0.15 to about 1.5 percent volume by volume;

b) this enzyme solution being formed by dissolving in water containing a portion of the total nonionic surfactant, cholesterol oxidase to provide in the net solution cholesterol oxidase in a concentration of at least about 0.02 KIU/l,

the base solution being adjusted if required to provide a net solution having a pH of from about 5.5 to about 8.5.

2. The method of claim 1 in which there is added to the enzyme solution microbial cholesterol esterase in an amount to provide in the net solution cholesterol esterase in a concentration of at least about 0.07 KIU/l.

3. The method of claim 1 or 2 in which there is added a chromogen system for determination of hydrogen peroxide.

4. The method of claim 3 in which there is added to the base solution phenol in an amount sufficient to provide in the net solution phenol in a concentration of from about 8 to about 15 mM, and 4-aminoantipyrine, and in which there is added to the enzyme solution peroxidase, the amount of peroxidase and 4-aminoantipyrine being sufficient in the net solution to enable quantitative colormetric determination of the amount of hydrogen peroxide formed from oxidation of cholesterol.

5. The method of claim 4 in which there is added phenol in a concentration of from about 8 to about 35 mM, 4-aminoantipyrine in a concentration of from about 0.25 to about 0.35 mM, and peroxidase in a concentration of at least about 30 KIU/l.

6. The method of any one of the previous claims in which there is added a bacteriocide.

7. The method of claim 6 in which there is added 2,4 dichlorophenol in a concentration of up to about 1 mM.

8. The method of any one of the previous claims in which the nonionic surfactant is polyethylene glycol p-isooctylphenyl ether.

9. The method of any one of the previous claims in which the buffer is potassium dihydrogen phosphate.

10. The method of any one of the previous claims in which the acidic compound is a metal salt of cholic acid.

11. A method for preparing a stable total cholesterol chromogen assay composition comprising forming an aqueous solution having a pH of from about 6.5 to about 8.5 by combining:

a) phenol in a concentration of from about 8 to about 35 mM;

b) a metal salt of cholic acid present in a concentration of up to about 5 mM;

c) a nonionic surfactant present in a concentration of from about 0.2 to about 1.5 percent volume by volume;

d) a buffer present in a concentration of from 0 to about 65 mM;

e) 4-aminoantipyrine;

f) microbial cholesterol esterase present in a concentration of at least about 0.07 KIU/l;

g) cholesterol oxidase present in a concentration of at least about 0.02 KIU/l; and

h) peroxidase,

the amount of peroxidase and 4-aminoantipyrine being sufficient to enable quantitative determination of the amount of hydrogen peroxide formed from oxidation of cholesterol.

12. The method of claim 11 in which the buffer is potassium dihydrogen phosphate and pH is from about 6 to about 7.5.

13. The method of Claim 11 or 12 in which the nonionic surfactant is present in a concentration of from about 0.2 to about 0.4 percent volume by volume and is polyethylene glycol p-isooctylphenyl ether.

14. The method of any one of the previous claims in which peroxidase is present in a concentration of at least about 30 KIU/l and in which 4-aminoantipyrine is present in a concentration of about 0.3 mM.

15. A method for preparing a stable total cholesterol chromogen assay composition comprising forming an aqueous solution by combining:

a) phenol in a concentration of about 17 mM;

b) 2,4 dichlorophenol present in a concentration of about 0.5 mM;

c) a metal salt of cholic acid present in a concentration of up to about 5 mM;

d) polyethylene glycol p-isooctylphenyl ether present in a concentration of from about 0.2 to about 0.6 percent volume by volume;

e) $KH_2PO_4$ present in a concentration of about 12.5 mM;

f) peroxidase present in a concentration of about 30 KIU/l;

g) cholesterol oxidase present in a concentration of at least about 0.05 KIU/l;

h) microbial cholesterol esterase present in a concentration of at least about 0.1 KIU/l; and

i) 4-aminoantipyrene present in a concentration of about 0.3 mM,

said stable total cholesterol chromogen assay composition having a pH of from about 6.0 to about 7.5.

**Patentansprüche für die Vertragsstaaten: BE, GB, FR, DE, NL, IT, LU, SE, CH, LI**

1. Stabile Zusammensetzung für den Cholesterin-Nachweis, welche eine wäßrige Lösung aus

a) wenigstens einer sauren Komponente, gewählt aus einer Gallensäure und einem Gallensäuresalz, wobei die saure Komponente in einer Gesamtmenge von bis zu etwa 5 mMol vorliegt,

b) einem nichtionischen, oberflächenaktiven Mittel, das in einer Konzentration von etwa 0,15 bis etwa 1,5% bezogen auf das Volumen, vorliegt,

c) einem Puffer in einer Konzentration von 0 bis etwa 65 mMol, und

d) Cholesterinoxidase in einer Konzentration von wenigstens etwa 0,02 KIU/l umfaßt,

wobei die Lösung zum Cholesterin-Nachweis einen pH von etwa 5,5 bis etwa 8,5 besitzt.

2. Zusammensetzung nach Anspruch 1, welche Mikrobencholesterinesterase in einer Konzentration von wenigstens etwa 0,07 KIU/l einschließt.

3. Zusammensetzung nach Anspruch 1 oder 2, welche ein chromogenes System zur Bestimmung von Wasserstoffperoxid einschließt.

4. Stabiles System für den Cholesterin-Nachweis nach Anspruch 3, worin das chromogene System Phenol in einer Konzentration von etwa 8 bis etwa 35 mMol und Peroxidase und 4-Aminoantipyrin in einer Konzentration, die ausreichend ist, um eine kolorimetrische, quantitative Bestimmung des durch die Oxidation von Cholesterin gebildeten Wasserstoffperoxids zur Verfügung zu stellen, umfaßt.

5. System nach Anspruch 1 oder 2, welches ein chromogenes System zur Bestimmung von Wasserstoffperoxid einschließt und welches Phenol in einer Konzentration von etwa 8 bis etwa 35 mMol, 4-Aminoantipyrin in einer Konzentration von etwa 0,25 bis etwa 0,35 mMol und Peroxidase in einer Konzentration von wenigstens etwa 30 KIU/l umfaßt.

6. Zusammensetzung nach einem der vorhergehenden Ansprüche, welche ein Bakterizid einschließt.

7. Zusammensetzung nach Anspruch 6, welche 2,4-Dichlorphenol in einer Konzentration von bis zu etwa 1 mMol einschließt.

8. Zusammensetzung nach einem der vorhergehenden Ansprüche, worin das nichtionische, oberflächenaktive Mittel Polyethylenglykol-p-isooctylphenylether ist.

9. Zusammensetzung nach einem der vorhergehenden Ansprüche, worin der Puffer Kaliumdihydrogenphosphat ist.

10. Zusammensetzung nach einem der vorhergehenden Ansprüche, worin die saure Komponente ein Metallsalz von Cholsäure ist.

11. Stabile Zusammensetzung für den Gesamt-Cholesterinchromogen-Nachweis, umfassend eine wäßrige Lösung mit einem pH von etwa 6,5 bis etwa 8,5 und umfassend

a) Phenol in einer Konzentration von etwa 8 bis etwa 35 mMol,

b) ein Metallsalz von Cholsäure in einer Konzentration von bis zu 5 mMol,

c) ein nichtionisches, oberflächenaktives Mittel in einer Konzentration von etwa 0,2 bis etwa 1,5%, bezogen auf das Volumen,

d) einen Puffer in einer Konzentration von 0 bis etwa 65 mMol,

e) 4-Aminoantipyrin,

f) Mikrobencholesterinesterase in einer Konzentration von wenigstens etwa 0,07 KIU/l,

g) Cholesterinoxidase in einer Konzentration von wenigstens etwa 0,02 KIU/l und

h) Peroxidase,

wobei die Menge an Peroxidase und 4-Aminoantipyrin ausreichend ist, um eine quantitative Bestimmung der Menge an Wasserstoffperoxid, die sich durch Oxidation von Cholesterin gebildet hat, zu ermöglichen.

12. Zusammensetzung nach Anspruch 11, worin der Puffer Kaliumdihydrogenphosphat ist und der pH etwa 6 bis etwa 7,5 beträgt.

13. Zusammensetzung nach Anspruch 11 oder 12, worin das nichtionische, oberflächenaktive Mittel in einer Konzentration von etwa 0,2 bis etwa 0,4%, bezogen auf das Volumen, vorliegt und Polyethylenglykol-p-isooctylphenylether ist.

14. Zusammensetzung nach einem der vorhergehenden Ansprüche, worin Peroxidase in einer Konzentration von wenigstens etwa 30 KIU/l vorliegt und worin 4-Aminoantipyrin in einer Konzentration von etwa 0,3 mMol vorliegt.

15. Stabile Zusammensetzung für den Gesamt-Cholesterinchromogen-Nachweis, umfassend eine wäßrige Lösung aus

a) Phenol in einer Konzentration von etwa 17 mMol,

b) 2,4-Dichlorphenol in einer Konzentration von etwa 0,5 mMol,

c) einem Metallsalz von Cholsäure in einer Konzentration von bis zu etwa 5 mMol,

d) Polyethylenglykol-p-isooctylphenylether in einer Konzentration von etwa 0,2 bis etwa 0,6%, bezogen auf das Volumen,

e) $KH_2PO_4$ in einer Konzentration von etwa 12,5 mMol,

f) Peroxidase in einer Konzentration von etwa 30 KIU/l,

g) Cholesterinoxidase in einer Konzentration von wenigstens etwa 0,05 KIU/l,

h) Mikrobencholesterinesterase in einer Konzentration von wenigstens etwa 0,1 KIU/l und

i) 4-Aminoantipyrin in einer Konzentration von etwa 0,3 mMol,

wobei die stabile Zusammensetzung für den Gesamt-Chosterinchromogen-Nachweis einen pH von etwa 6,0 bis etwa 7,5 besitzt.

16. Verfahren zur Herstellung einer stabilen Lösung für den Cholesterin-Nachweis, bei dem eine basische Lösung und eine Enzymlösung gebildet werden, dann die basische Lösung und die Enzymlösung zur Bildung einer Mischlösung kombiniert werden, und worin

a) die basische Lösung durch Lösen in Wasser von

i) wenigstens einer sauren Komponente, gewählt aus einer Gallensäure und einem Gallensäuresalz, um eine Gesamtmenge der sauren Komponente in der Mischlösung von bis zu etwa 5 mMol zur Verfügung zu stellen,

ii) eines nichtionischen, oberflächenaktiven Mittels, welches in einer Konzentration von etwa 0,15 bis etwa 1,5%, bezogen auf das Volumen, in der Mischlösung vorliegt,

gebildet wird,

b) die Enzymlösung durch Lösen in Wasser, enthaltend einen Teil des gesamten nichtionischen, oberflächenaktiven Mittels, Cholesterinoxidase, um in der Mischlösung Cholesterinoxidase in einer Konzentration von wenigstens etwa 0,02 KIU/l zur Verfügung zu stellen, gebildet wird,

wobei die basische Lösung, wenn notwendig, eingestellt wird, um eine Mischlösung mit einem pH von etwa 5,5 bis etwa 8,5 zur Verfügung zu stellen.

17. Verfahren nach Anspruch 16, worin der Enzymlösung Mikrobencholesterinesterase in einer Menge zugegeben wird, um in der Mischlösung Cholesterinesterase in einer Konzentration von wenigstens etwa 0,07 KIU/l zur Verfügung zu stellen.

18. Verfahren nach Anspruch 16 oder 17, worin der basischen Lösung Phenol in einer Menge, die ausreichend ist, um in der Mischlösung Phenol in einer Konzentration von etwa 8 bis etwa 15 mMol zur Verfügung zu stellen, und 4-Aminoantipyrin zugegeben wird, und worin der Enzymlösung Peroxidase zugegeben wird, wobei die Menge an Peroxidase und 4-Aminoantipyrin in der Mischlösung ausreichend

ist, um eine kolometrische Bestimmung der Menge an durch Oxidation des Cholesterins gebildeten Wasserstoffperoxids zu ermöglichen.

**Patentansprüche für den Vertragsstaat: AT**

1. Verfahren zur Herstellung einer stabilen Lösung für den Cholesterin-Nachweis, bei den eine basische Lösung und eine Enzymlösung gebildet werden, dann die basische Lösung und die Enzymlösung zur Bildung einer Mischlösung kombiniert werden, und worin

a) die basische Lösung durch Lösen in Wasser von

i) wenigstens einer sauren Verbindung, gewählt aus einer Gallensäure und einem Gallensäuresalz, um eine Gesamtmenge der sauren Komponente in der Mischlösung von bis zu etwa 5 mMol zur Verfügung zu stellen,

ii) eines nichtionischen, oberflächenaktiven Mittels, um in der Mischlösung eine Konzentration des nichtionischen, oberflächenaktiven Mittels von etwa 0,15 bis etwa 1,5%, bezogen auf das Volumen, zur Verfügung zu stellen,

gebildet wird,

b) die Enzymlösung durch Lösen in Wasser, enthaltend einen Teil des gesamten nichtionischen, oberflächenaktiven Mittels, Cholesterinoxidase, um in der Mischlösung Cholesterinoxidase in einer Konzentration von wenigstens etwa 0,02 KIU/l zur Verfügung zu stellen, gebildet wird.

wobei die basische Lösung, wenn notwendig, eingestellt wird, um eine Mischlösung mit einem pH von etwa 5,5 bis etwa 8,5 zur Verfügung zu stellen.

2. Verfahren nach Anspruch 1, worin der Enzymlösung Mikrobencholesterinesterase in einer Menge zugegeben wird, um in der Mischlösung Cholesterinesterase in einer Konzentration von wenigstens etwa 0,07 KIU/l zur Verfügung zu stellen.

3. Verfahren nach Anspruch 1 oder 2, worin ein chromogenes System zur Bestimmung von Wasserstoffperoxid zugegeben wird.

4. Verfahren nach Anspruch 3, worin der basischen Lösung Phenol in einer Menge, die ausreichend ist, um in der Mischlösung Phenol in einer Konzentration von etwa 8 bis etwa 15 mMol zur Verfügung zu stellen, und 4-Aminoantipyrin zugegeben wird, und worin der Enzymlösung Peroxidase zugegeben wird, wobei die Menge der Peroxidase und des 4-Aminoantipyrins in der Mischlösung ausreichend ist, um eine quantitative, kolorimetrische Bestimmung der Menge des durch Oxidation von Cholesterin gebildeten Wasserstoffperoxids zu ermöglichen.

5. Verfahren nach Anspruch 4, worin Phenol in einer Konzentration von etwa 8 bis etwa 35 mMol, 4-Aminoantipyrin in einer Konzentration von etwa 0,25 bis etwa 0,35 mMol und Peroxidase in einer Konzentration von wenigstens etwa 30 KIU/l zugegeben werden.

6. Verfahren nach einem der vorhergehenden Ansprüche, worin ein Bakterizid zugegeben wird.

7. Verfahren nach Anspruch 6, worin 2,4-Dichlorphenol in einer Konzentration von bis zu etwa 1 mMol zugegeben wird.

8. Verfahren nach einem der vorhergehenden Ansprüche, worin das nichtionische, oberflächenaktive Mittel Polyethylenglykol-p-isooctylphenylether ist.

9. Verfahren nach einem der vorhergehenden Ansprüche, worin der Puffer Kaliumdihydrogenphosphat ist.

10. Verfahren nach einem der vorhergehenden Ansprüche, worin die saure Verbindung ein Metallsalz von Cholsäure ist.

11. Verfahren zur Herstellung einer stabilen Zusammensetzung für den Gesamt-Cholesterinchromogen-Nachweis, bei dem eine wäßrige Lösung mit einem pH von etwa 6,5 bis etwa 8,5 gebildet wird durch Kombinieren von

a) Phenol in einer Konzentration von etwa 8 bis etwa 35 mMol,

b) eines Metallsalzes von Cholsäure in einer Konzentration von bis zu etwa 5 mMol,

c) eines nichtionischen, oberflächenaktiven Mittels in einer Konzentration von etwa 0,2 bis etwa 1,5%, bezogen auf das Volumen,

d) eines Puffers in einer Konzentration von 0 bis etwa 65 mMol,

e) 4-Aminoantipyrin,

f) Mikrobencholesterinesterase in einer Konzentration von wenigstens etwa 0,07 KIU/l,

g) Cholesterinoxidase in einer Konzentration von wenigstens etwa 0,02 KIU/l und

h) Peroxidase,

wobei die Menge der Peroxidase und des 4-Aminoantipyrins ausreichend ist, um eine quantitative Bestimmung der Menge des durch Oxidation von Cholesterin gebildeten Wasserstoffperoxids zu ermöglichen.

12. Verfahren nach Anspruch 11, worin der Puffer Kaliumdihydrogenphosphat ist und der pH etwa 6 bis etwa 7,5 beträgt.

13. Verfahren nach Anspruch 11 oder 12, worin der nichtionische, oberflächenaktive Mittel in einer

Konzentration von etwa 0,2 bis etwa 0,4%, bezogen auf das Volumen, vorliegt und Polyethylenglykol-p-isooctylphenylether ist.

14. Verfahren nach einem der vorhergehenden Ansprüche, worin Peroxidase in einer Konzentration von wenigstens etwa 30 KIU/l vorliegt und worin 4-Aminoantipyrin in einer Konzentration von etwa 0,3 mMol vorliegt.

15. Verfahren zur Herstellung einer stabilen Zusammensetzung für den Gesamt-Cholesterinchromogen-Nachweis, bei dem eine wäßrige Lösung gebildet wird durch Kombinieren

a) von Phenol in einer Konzentration von etwa 17 mMol,

b) von 2,4-Dichlorphenol in einer Konzentration von etwa 0,5 mMol,

c) eines Metallsalzes von Cholsäure in einer Konzentration von bis zu etwa 5 mMol,

d) von Polyethylenglykol-p-isooctylphenylether in einer Konzentration von etwa 0,2 bis etwa 0,6%, bezogen auf das Volumen,

e) von $KH_2PO_4$ in einer Konzentration von etwa 12,5 mMol,

f) von Peroxidase in einer Konzentration von etwa 30 KIU/l,

g) von Cholesterinoxidase in einer Konzentration von wenigstens etwa 0,05 KIU/l,

h) von Mikrobencholesterinesterase in einer Konzentration von wenigstens etwa 0,1 KIU/l und

i) von 4-Aminoantipyrin in einer Konzentration von etwa 0,3 mMol,

wobei die stabile Zusammensetzung für den Gesamt-Cholesterinchromogen-Nachweis einen pH von etwa 6,0 bis etwa 7,5 besitzt.

**Revendications pour les Etats Contractants: BE, CH, DE, FR, GB, IT, LI, LU, NL, SE**

1. Composition de dosage du cholestérol stable, qui comprend une solution aqueuse:

(a) d'au moins un composé acide choisi parmi un acide biliaire ou un sel d'acide biliaire, le total de ce composé acide étant présent dans une quantité allant jusqu'à environ 5 mM;

(b) un agent tensioactif non ionique présent à une concentration allant de 0,15 à environ 1,5% en volume;

(c) un tampon à une concentration de 0 à environ 65 mM; et

(d) de la cholestérol oxydase à une concentration d'au moins environ 0,02 KUI/l,

cette solution de dosage du cholestérol ayant un pH d'environ 5,5 à environ 8,5.

2. Composition de dosage du cholestérol stable suivant la revendication 1, qui contient de la cholestérol estérase microbienne présente à une concentration d'au moins environ 0,07 KUI/l.

3. Composition de dosage du cholestérol stable suivant les revendications 1 ou 2, qui contient un système chromogène pour le dosage du péroxyde d'hydrogène.

4. Système de dosage du cholestérol stable suivant la revendication 3, dans lequel le système chromogène comprend du phénol à une concentration d'environ 8 à environ 35 mM, et de la péroxydase et de la 4-aminoantipyrine à une concentration suffisante pour donner un dosage colorimétrique quantitatif du peroxyde d'hydrogène formé à partir de l'oxydation du cholestérol.

5. Système de dosage du cholestérol stable suivant la revendication 1 ou 2, qui contient un système chromogène pour doser le peroxyde d'hydrogène et qui comprend du phénol à une concentration d'environ 8 à environ 35 mM, de la 4-aminoantipyrine à une concentration d'environ 0,25 à environ 0,35 mM, et de la peroxydase à une concentration d'au moins 30 KUI/l.

6. Composition de dosage du cholestérol stable suivant l'une quelconque des revendications précédentes, qui contient un bactériocide.

7. Composition de dosage du cholestérol stable suivant la revendication 6, qui contient du 2,4-dichlorophénol à une concentration allant jusqu'à environ 1 mM.

8. Composition du dosage du cholestérol stable suivant l'une quelconque des revendications précédentes, dans laquelle l'agent tensioactif non ionique est un éther p-isooctylphénylique du polyéthylèneglycol.

9. Composition du dosage du cholestérol stable suivant l'une quelconque des revendications précédentes, dans laquelle le tampon est du dihydrogénophosphate de potassium.

10. Composition de dosage du cholestérol stable suivant l'une quelconque des revendications précédentes, dans laquelle le composé acide est un sel métallique de l'acide cholique.

11. Composition de dosage chromogène du cholestérol total stable comprenant une solution aqueuse ayant un pH d'environ 6,5 à environ 8,5, et comprenant:

(a) du phénol à une concentration d'environ 8 à environ 35 mM,

(b) un sel métallique de l'acide cholique présent à une concentration allant jusqu'à environ 5 mM;

(c) un agent tensioactif non ionique présent à une concentration d'environ 0,2 à environ 1,5% en volume;

(d) un tampon présent à une concentration d'environ 0 à environ 65 mM;

(e) de la 4-aminoantipyrine;

(f) de la cholestérol estérase microbienne présente à une concentration d'au moins environ 0,07 KUI/l;

(g) de la cholestérol estérase présente à une concentration d'au moins environ 0,02 KUI/l; et

(h) de la peroxydase.

la quantité de peroxydase et de 4-aminoantipyrine étant suffisante pour permettre une détermination quantitative de la quantité de peroxyde d'hydrogène formée par oxydation du cholestérol.

12. Composition de dosage chromogène du cholestérol total stable suivant la revendication 11, dans laquelle le tampon est du dihydrogénophosphate de potassium, et le pH est d'environ 6 à environ 7,5.

13. Composition de dosage chromogène du cholestérol total stable suivant les revendications 11 ou 12, dans laquelle l'agent tensioactif non ionique est présent à une concentration d'environ 0,2 à environ 0,4% en volume et est de l'éther p-isooctylphénylique du polyéthylène glycol.

14. Composition de dosage chromogène du cholestérol total stable suivant l'une quelconque des revendications précédentes, dans laquelle le peroxydase est présent à une concentration d'au moins environ 30 KUI/l et dans laquelle la 4-aminoantipyrine est présente à une concentration d'environ 0,3 mM.

15. Composition de dosage chromogène du cholestérol total stable comprenant une solution aqueuse:

(a) d'un phénol à une concentration d'environ 17 mM;

(b) du 2,4-dichlorophénol présent à une concentration d'environ 0,5 mM;

(c) d'un sel métallique de l'acide cholique présent à une concentration allant jusqu'à environ 5 mM;

(d) d'éther p-isooctylphénylique du polyéthylène glycol présent à une concentration d'environ 0,2 à environ 0,6% en volume;

(e) de $KH_2PO_4$ présent à une concentration d'environ, 12,5 mM;

(f) de peroxydase présent à une concentration d'environ 30 KUI/l;

(g) de cholestérol oxydase présent à une concentration d'au moins environ 0,05 KUI/l;

(h) de cholestérol estérase microbienne présent à une concentration d'au moins environ 0,1 KUI/l; et

(i) de 4-aminoantipyrine présent à une concentration d'environ 0,3 mM;

cette composition de dosage chromogène du cholestérol total stable ayant un pH d'environ 6,0 à environ 7,5.

16. Procédé de préparation d'une solution de dosage du cholestérol stable, qui consiste à former une solution de base et une solution d'enzyme, puis à combiner la solution de base et la solution d'enzymes pour former une solution finale et dans lequel:

(a) la solution de base est formée en dissolvant dans de l'eau:

1— au moins un composé acide choisi parmi un acide biliaire et un sel d'un acide biliaire pour donner un total de ce composé acide dans la solution finale dans une quantité allant jusqu'à environ 5 mM;

2— un agent tensioactif donnant dans la solution finale une concentration d'agents tensioactifs non ionique d'environ 0,15 à environ 1,5% en volume;

(b) la solution d'enzyme étant formée en dissolvant dans de l'eau contenant une partie de l'agent tensioactif non nionique total, de la cholestérol oxydase pour donner dans la solution finale une concentration en cholestérol oxydase d'au moins environ 0,02 KUI/l,

la solution de base étant ajustée si nécessaire pour donner une solution finale ayant un pH d'environ 5,5 à environ 8,5.

17. Procédé suivant la revendication 16, dans lequel on ajoute à la solution d'enzyme de la cholestérol estérase microbienne dans une quantité donnant dans la solution finale de la cholestérole estérase à une concentration d'au moins environ 0,07 KUI/l.

18. Procédé suivant les revendications 16 ou 17, dans lequel on ajoute à la solution de base du phénol dans une quantité suffisante pour donner dans la solution finale du phénol à une concentration d'environ 8 à environ 15 mM et de la 4-aminoantipyrine, et dans lequel on ajoute à la solution d'enzyme de la peroxydase, la quantité de peroxydase et de 4-aminoantipyrine et étant suffisante dans la solution finale pour permettre une détermination colorimétrique quantitative de la quantité de peroxyde d'hydrogène formée par oxydation du cholestérol.

**Revendications pour l'Etat Contractant: AT**

1. Procédé de préparation d'une solution de dosage du cholestérol stable, qui consiste à former une solution de base et une solution d'enzyme, puis à combiner la solution de base et la solution d'enzyme pour former une solution finale, et dans lequel:

(a) la solution de base est formée en dissolvant dans de l'eau:

1— au moins un composé acide choisi parmi un acide biliaire et un sel d'un acide biliaire pour donner un total de ce composé acide dans la solution finale allant jusqu'à environ 5 mM;

2— un agent tensioactif dans une quantité telle qu'il donne dans la solution finale une concentration en agents tensioactifs non ioniques d'environ 0,15 à environ 1,5% en volume,

(b) la solution d'enzyme étant formée en dissolvant dans de l'eau contenant une partie de l'agent tensioactif non ionique total, de la cholestérol oxydase pour donner dans la solution finale une concentration en cholestérol oxydase d'au moins environ 0,02 KUI/l, la solution de base étant ajustée si nécessaire pour donner une solution finale ayant un pH d'environ 5,5 à environ 8,5.

2. Procédé suivant la revendication 1, dans lequel on ajoute à la solution d'enzyme de la cholestérol

estérase microbienne dans une quantité donnant dans la solution finale une concentration en cholestérol estérase d'au moins environ 0,07 KUI/l.

3. Procédé suivant les revendications 1 ou 2, dans lequel on ajoute un système chromogène pour le dosage du peroxyde d'hydrogène.

4. Procédé de la revendication 3, dans lequel on ajoute à la solution de base du phénol dans une quantité suffisante pour donner dans la solution finale du phénol à une concentration d'environ 8 à environ 15 mM, et de la 4-aminoantipyrine, et dans lequel on ajoute à la solution d'enzyme de la peroxydase, la quantité de peroxydase et de 4-aminoantipyrine étant suffisante dans la solution finale pour permettre une détermination colorimétrique quantitative de la quantité de peroxyde d'hydrogène formée par oxydation du cholestérol.

5. Procédé de la revendication 4, dans lequel on ajoute du phénol à une concentration d'environ 8 à environ 35 mM, de la 4-aminoantipyrine à une concentration à environ 0,25 à environ 0,35 mM, et de la peroxydase à une concentration d'au moins environ 30 KUI/l.

6. Procédé suivant l'une quelconque des revendications précédentes dans lequel on ajoute un bactériocide.

7. Procédé de la revendication 6, dans lequel on ajoute de 2,4-dichlorophénol à une concentration allant jusqu'à environ 1 mM.

8. Procédé suivant l'une quelconque des revendications précédentes, dans lequel l'agent tensioactif non ionique est de l'éther p-isooctylphénylique du polyéthylène glycol.

9. Procédé suivant l'une quelconque des revendications précédentes, dans lequel le tampon est du dihydrogénophosphate de potassium.

10. Procédé suivant l'une quelconque des revendications précédentes, dans lequel le composé acide est un sel métallique de l'acide cholique.

11. Procédé de préparation d'une composition de dosage chromogène du cholestérol total stable, consistant à former une solution aqueuse ayant un pH d'environ 6,5 à environ 8,5, en combinant:

(a) phénol à une concentration d'environ 8 à environ 35 mM;

(b) un sel métallique de l'acide cholique présent à une concentration allant jusqu'à environ 5 mM;

(c) un agent tensioactif non ionique présent à une concentration d'environ 0,2 à environ 1,5% en volume;

(d) un tampon présent à une concentration d'environ 0 à environ 65 mM;

(e) de la 4-aminoantipyrine;

(f) de la cholestérol estérase microbienne présente à une concentration d'au moins environ 0,07 KUI/l;

(g) de la cholestérol estérase présente à une concentration d'au moins environ 0,02 KUI/l;

(h) de la peroxydase,

la quantité de peroxydase et de 4-aminoantipyrine étant suffisante pour permettre une détermination quantitative de la quantité de peroxyde d'hydrogène formée par oxydation du cholestérol.

12. Procédé de la revendication 11, dans lequel le tampon est du dihydrogénophosphate de potassium et le pH est d'environ 6 à environ 7,5.

13. Procédé suivant les revendications 11 ou 12, dans lequel l'agent tensioactif non ionique est présent à une concentration d'environ 0,2 à environ 0,4% en volume et est de l'éther p-isooctylphénylique du polyéthylène glycol.

14. Procédé suivant l'une quelconque des revendications précédentes, dans lequel la peroxydase est présente à une concentration d'au moins environ 30 KUI/l et dans laquelle la 4-aminoantipyrine est présente à une concentration d'environ 0,3 mM.

15. Procédé de préparation d'une composition de dosage chromogène du cholestérol total stable, consistant à former une solution aqueuse en combinant:

(a) du phénol à une concentration d'environ 17 mM;

(b) du 2,4-dichlorophénol présent à une concentration d'environ 0,5 mM;

(c) un sel métallique de l'acide cholique présent à une concentration allant jusqu'à environ 5 mM;

(d) de l'éther p-isooctylphénylique du polyéthylène glycol présent à une concentration d'environ 0,2 à environ 0,6% en volume;

(e) du $KH_2PO_4$ présent à une concentration d'environ 12,5 mM;

(f) de la peroxydase présente à une concentration d'environ 30 KUI/l;

(g) de la cholestérol oxydase présente à une concentration d'au moins environ 0,05 KUI/l;

(h) de la cholestérol estérase microbienne présente à une concentration d'au moins environ 0,1 KUI/l; et

(i) de la 4-aminoantipyrine présente à une concentration d'environ 0,3 mM;

cette composition de dosage chromogène du cholestérol total stable ayant un pH d'environ 6,0 à environ 7,5.